# EUROPEAN PATENT APPLICATION

(11) **EP 4 497 446 A1**
(43) Date of publication of application: **29.01.2025**
(21) Application number: 23194430.7
(22) Date of filing: 31.08.2023
(51) Int. Cl.: A61L 2/08, A45D 27/46, B26B 19/38

(54) **LIGHT-BASED DISINFECTING DEVICE FOR A PERSONAL CARE DEVICE WITH DRYING FUNCTION**

(30) Priority: 27.07.2023 WO PCT/CN2023/109543
(71) Applicant: Koninklijke Philips N.V., 5656 AG Eindhoven (NL)
(72) Inventor: LELIEVELD, Mark Johannes, 5656AG Eindhoven (NL); VAN ABEELEN, Frank Anton, 5656AG Eindhoven (NL); VAN DER ZWAN, Eduard Antonius, 5656AG Eindhoven (NL); ZHANG, Jun, 5656AG Eindhoven (NL); TAN, Liangwei, 5656AG Eindhoven (NL)
(74) Representative: Philips Intellectual Property & Standards

(57) **Abstract**

The invention provides a disinfecting device (5) for disinfecting a personal care device (3) by disinfecting light. The disinfecting device comprises a housing (17) wherein a light module (23) is arranged which has a light source (25), a first main side (27) emitting the disinfecting light generated by the light source, and a second main side (29) opposite to the first main side. The housing (17) further accommodates a receiving chamber (49), arranged at the first main side of the light module to hold at least a portion of the personal care device in a disinfecting position, and an air chamber (51) arranged at the second main side of the light module. At least one first air channel (59a, 59b) connects the receiving space with the air chamber, and at least one second air channel (61) connects the air chamber with the ambient space. Each first air channel is arranged exclusively outside of the light module, and the disinfecting device is configured to provide, in the disinfecting position of said portion of the personal care device, at least one third air channel (63) connecting the receiving chamber with the ambient space. During use, the light source heats the air in the air chamber and the receiving chamber, thus creating a natural-convection air flow through the disinfecting device enabling efficient drying of said portion of the personal care device in addition to the disinfecting function.

## Description

### FIELD OF THE INVENTION

The invention relates to a personal care system comprising a personal care device and a disinfecting device, wherein the personal care device comprises a personal care treatment unit configured to provide a personal care treatment to a body part of a subject, wherein the disinfecting device is configured to disinfect the personal care treatment unit by means of disinfecting light, and wherein the disinfecting device comprises:
- a housing;
- a light module arranged in the housing and comprising at least one light source configured to generate the disinfecting light, a first main side via which during use the disinfecting light generated by the at least one light source is emitted, and a second main side opposite to the first main side;
- a receiving space arranged at the first main side of the light module and configured to receive at least a portion of the personal care treatment unit;
- an air chamber arranged in the housing at the second main side of the light module;
- at least one first air channel connecting the receiving space with the air chamber; and
- at least one second air channel connecting the air chamber with ambient space surrounding the housing.

The invention further relates to a disinfecting device configured to disinfect a personal care treatment unit of a personal care device by means of disinfecting light, wherein the personal care treatment unit is configured to provide a personal care treatment to a body part of a subject, and wherein the disinfecting device comprises:
- a housing;
- a light module arranged in the housing and comprising at least one light source configured to generate the disinfecting light, a first main side via which during use the disinfecting light generated by the at least one light source is emitted, and a second main side opposite to the first main side;
- a receiving space arranged at the first main side of the light module and configured to receive at least a portion of the personal care treatment unit;
- an air chamber arranged in the housing at the second main side of the light module;
- at least one first air channel connecting the receiving space with the air chamber; and
- at least one second air channel connecting the air chamber with ambient space surrounding the housing.

### BACKGROUND OF THE INVENTION

Personal care systems and devices configured to provide a personal care treatment to a body part of a subject are widely known. Examples are electric shavers to shave beard hairs on the face of a human, hair-trimming devices to cut beard hairs on the face of a human or hairs on human body parts other than the face, intense pulsed light (IPL) devices to remove hairs on the legs or other body parts of a human, facial-cleansing brushing devices to clean the face of a human by means of a rotating and/or vibrating brush, and skin rejuvenation devices to rejuvenate the skin of body parts of a human by means of electromagnetic or radiofrequency energy. Such personal care devices usually have a main body and a personal care treatment unit coupled to the main body that actually provides the personal care treatment to the body part. After one or more treatment sessions with the personal care device, the personal care treatment unit of the personal care device may need to be cleaned and/or disinfected. In this respect, it is known to use a personal care device with a disinfecting device that generates ultraviolet, UV, disinfecting light to disinfect the personal care treatment unit.

KR20110126002A discloses an electric shaver having a main body and a shaving head arranged on the main body. The shaver further has a shaving-head cap that can be releasably coupled to an upper portion of the main body to cover and protect the shaving head, e.g., when the shaver is not in use. The shaving-head cap is provided with a sterilization module including a plurality of UV LEDs which are arranged to irradiate the shaving head with UV light when the shaving-head cap is coupled to the main body and covers the shaving head. The UV LEDs are powered by means of a power source arranged in the main body, so that the UV LEDs can be activated only when the shaving-head cap is correctly coupled to the main body. In this way any leakage of UV light to the environment, which may be harmful for the user, is prevented. The UV LEDs are activated for a predetermined period of time to sufficiently sterilize the shaving head by irradiation with the UV light. A disadvantage of this known disinfecting device is that, in case the shaving head is wet, the shaving head will not be dried during the sterilization process, because it is fully enclosed by the shaving-head cap during the sterilization process. This may result in unwanted odor.

KR20200111078A discloses a charging device for an electric shaver, comprising a UV light module arranged to irradiate the shaving unit of the shaver with UV light when the shaver is arranged in the charging device for being charged. Thus, during charging of the shaver, the shaving unit of the shaver can be sterilized by the UV light emitted by the UV light module. The charging device comprises a base portion on which the shaver can be placed in an upright position. The base portion is provided with electric charging contacts which are connectable with electric charging contacts provided in a lower part of the main body of the shaver when the shaver is correctly placed in the charging device. The UV light module is arranged in an upper housing portion of the charging device, which is connected to the base portion via a vertically extending column and positioned vertically above the base portion. The shaver is placeable in an open receiving space between the base portion and the upper housing portion of the charging device, so that the shaving unit of the shaver can be irradiated by the UV light emitted by the UV light module in the upper housing part. The UV light module comprises a circuit board, a plurality of UV LEDs mounted on a lower surface of the circuit board facing the receiving space, and a diffusive transparent cover arranged between the circuit board and the receiving space. The upper housing portion further has an air chamber arranged above the circuit board. The air chamber is connected with the receiving space via a plurality of air holes provided in the circuit board and a plurality of air holes provided in the diffusive transparent cover. The air chamber is connected with the environment by means of a plurality of air supply holes provided in a top wall of the upper housing portion. A blowing fan is arranged in the air chamber. During the sterilization process, with the UV LEDs emitting the UV light, the blowing fan is activated to suck ambient air via the air supply holes into the air chamber and to blow the air towards the shaving unit in the receiving space via the air holes provided in the circuit board and in the diffusive transparent cover. Since the UV LEDs will be heated as a result of their limited electric-to-optical energy conversion efficiency, the air which is blown towards the shaving unit will be heated when passing the air chamber and the air holes in the circuit board. As a result, a warm air flow is generated towards the shaving unit, which will increase the temperature of the shaving unit. The increased temperature of the shaving unit is intended to increase the shaving comfort experienced by the user during a next shaving session. A disadvantage of this known disinfecting device is that the blowing fan complicates the structure of the disinfecting device. Furthermore, the air flow through the air holes in the circuit board may pollute the electric components arranged on the circuit board, and the air holes in the diffusive transparent cover may reduce the uniformity of the light intensity provided by the diffusive transparent cover. Furthermore, the flow path of the ambient air through the UV light module is relatively short, so that the temperature increase of the air is limited.

### SUMMARY OF THE INVENTION

It is an object of the present invention to provide a personal care system and a disinfecting device, each having the features as mentioned in the section "field of the invention" here before, by means of which the disadvantages associated with the known disinfecting devices as described here before in the section "background of the invention" may at least be alleviated. In particular, an object of the present invention is to provide a personal care system and a disinfecting device wherein, in the disinfecting device, an air flow is generated along the personal care treatment unit without the use of a fan or other type of mechanical air-pumping system and sufficient to dry the personal care treatment unit within reasonable time when the personal care treatment unit is wet when placed in the disinfecting device. A further object of the present invention is to more efficiently heat the air that flows through the disinfecting device using the heat generated by the light source of the light module.

According to a first aspect of the invention, to achieve the above-mentioned object, a personal care system having the features as mentioned in the section "field of the invention" here before is characterized in that the receiving space is provided in a receiving chamber which is arranged in the housing of the disinfecting device and configured to hold said portion of the personal care treatment unit in a disinfecting position within the receiving chamber, in that each first air channel is arranged exclusively outside of the light module, and in that, in the disinfecting position of the personal care treatment unit, at least one third air channel is provided connecting the receiving chamber with the ambient space surrounding the housing.

According to a second aspect of the invention, to achieve the above-mentioned object, a disinfecting device having the features as mentioned in the section "field of the invention" here before is characterized in that the receiving space is provided in a receiving chamber which is arranged in the housing of the disinfecting device and configured to hold said portion of the personal care treatment unit in a disinfecting position within the receiving chamber, in that each first air channel is arranged exclusively outside of the light module, and in that the disinfecting device is configured to provide, in the disinfecting position of the personal care treatment unit, at least one third air channel connecting the receiving chamber with the ambient space surrounding the housing.

In a disinfecting device according to the invention and in a personal care system having a disinfecting device according to the invention, during use of the disinfecting device, with the personal care treatment unit of the personal care device being arranged in the disinfecting position within the receiving chamber of the disinfecting device, both the air in the air chamber at the second main side of the light module and the air in the receiving chamber at the first main side of the light module will be heated by the heat which is generated by the at least one light source of the light module as a result of the limited electric-to-optical power conversion efficiency of the light source. The heated air in the air chamber and receiving chamber will generate an air flow from the air chamber to the receiving chamber or vice versa, depending on the orientation of the disinfecting device relative to earth's gravity.

In a first orientation wherein the air chamber is positioned above the receiving chamber, the heated air in the air chamber will flow to the ambient space via the at least one second air channel that connects the air chamber with the ambient space, and the heated air in the receiving chamber will flow to the air chamber via the at least one first air channel that connects the receiving chamber with the air chamber. As a result, fresh ambient air will be sucked into the receiving chamber via the at least one third air channel that connects the receiving chamber with the ambient space. This results in an overall air flow through the disinfecting device from the ambient space via, respectively, the third air channel, the receiving chamber, the first air channel, the air chamber and the second air channel back into the ambient space.

In a second orientation wherein the air chamber is positioned below the receiving chamber, the heated air in the receiving chamber will flow to the ambient space via the at least one third air channel that connects the receiving chamber with the ambient space, and the heated air in the air chamber will flow to the receiving chamber via the at least one first air channel that connects the air chamber with the receiving chamber. As a result, fresh ambient air will be sucked into the air chamber via the at least one second air channel that connects the air chamber with the ambient space. This results in an overall air flow through the disinfecting device from the ambient space via, respectively, the second air channel, the air chamber, the first air channel, the receiving chamber and the third air channel back into the ambient space.

Because, according to the invention, each first air channel, that connects the air chamber with the receiving chamber, is arranged exclusively outside of the light module, a relatively long flow path can be created along the first and second main sides of the light module for the air flow from the receiving chamber to the air chamber, i.e., in said first orientation of the disinfecting device, and for the air flow from the air chamber to the receiving chamber, i.e., in said second orientation of the disinfecting device. Said relatively long flow path results in an effective heating of the air in the air chamber and receiving chamber, i.e., a relatively high temperature of the air. As a result, both in said first orientation and in said second orientation a strong air flow can be generated in the disinfecting device which is only based on natural convection, i.e., without the use of forced convection by a fan, pump, suction system or similar means. Both in the first orientation and in the second orientation, said natural-convection air flow is sufficient to dry the personal care treatment unit in the receiving chamber within a reasonable time, i.e., in case the personal care treatment unit is wet when placed in the receiving chamber.

It will be clear for the skilled person that the flow path of the air flow in the air chamber will depend on the positions of the first air channel and the second air channel relative to the air chamber and that said positions can be selected in a straight-forward manner to create a desired flow-path length in the air chamber. Likewise, it will be clear for the skilled person that the flow path of the air flow in the receiving chamber will depend on the positions of the first air channel and the third air channel relative to the receiving chamber and that said positions can be selected in a straight-forward manner to create a desired flow-path length in the receiving chamber.

It is further noted that in said second orientation of the disinfecting device the temperature of the air flow along the personal care treatment unit in the receiving chamber will be higher than in said first orientation of the disinfecting device, so that in general in said second orientation the required drying time may be shorter than in said first orientation. Both in said first orientation and in said second orientation, the natural-convection air flow along the first and second main sides of the light module provides an efficient cooling of the light module.

In embodiments of the personal care system and the disinfecting device according to the invention, the light module comprises a carrier, the at least one light source is mounted on a first main surface of the carrier which faces the first main side of the light module, and the light module comprises a heat sink connected to a second main surface of the carrier opposite to the first main surface of the carrier. The carrier may be a plate-shaped carrier, such as a printed circuit board, PCB. In these embodiments the heat sink will absorb a largest portion of the heat generated by the at least one light source on the carrier. Because the heat sink is connected to the second main surface of the carrier which faces the second main side of the light module, the air in the air chamber at the second main side of the light module will be heated more than the air in the receiving chamber at the first main side of the light module. The resulting temperature difference between the air in the air chamber and the air in the receiving chamber will increase the natural-convection air flow in the disinfecting device.

In further embodiments of the personal care system and the disinfecting device according to the invention, the light module comprises a light-diffusing member arranged at the first main side of the light module. The light-diffusing member may be a plate-shaped member extending parallel to the first main side of the light module. The light-diffusing member may be made from an optically transparent diffusive material. A main side of the plate-shaped member, preferably a main side facing the at least one light source, may be provided with an optical structure comprising a plurality of facets. A main side of the plate-shaped member facing the receiving chamber may form the first main side of the light module. The angular distribution of the disinfecting light generated by the at least one light source is increased by the light-diffusing member, so that the disinfecting light is more uniformly distributed over the personal care treatment unit and any peak irradiances of the disinfecting light emitted at the first main side of the light module are limited.

In preferred embodiments of the personal care system and the disinfecting device according to the invention, the disinfecting light generated by the at least one light source has a peak wavelength in a range from 400 to 425 nm, preferably in a range from 405 to 410 nm. In these preferred embodiments the peak wavelength of the disinfecting light generated by the at least one light source is in the violet range of visible light. The effectiveness of violet light with a peak wavelength within the range from 400 to 425 nm for reducing bacteria like Escherichia coli, E. coli, and Staphylococcus aureus, S. aureus, is high, but violet light is significantly less harmful for the user as compared to UV light. As a result, in these preferred embodiments any safety measures to be provided in the disinfecting device to prevent unintentional exposure of the user to the disinfecting light might be less stringent compared with disinfecting devices that use UV light. In particular, it might be sufficient to provide the light module with a light-diffusing member to sufficiently reduce peak irradiances of the disinfecting light emitted by the light module, and it may not be necessary to provide any particular measures to prevent leakage of scattered disinfecting light via openings or gaps in the housing of the disinfecting device, such as the air channels that connect the air chamber and the receiving chamber with the ambient space. It is however noted that the invention also covers embodiments wherein the disinfecting light generated by the at least one light source has a peak wavelength in a range different from the range from 400 to 425 nm, such as UV light, light in the blue range of visible light, or light with a wavelength even longer than the wavelength of blue light.

In embodiments of the personal care system and the disinfecting device according to the invention, the light module comprises an outer circumferential surface extending between the first and second main sides of the light module, and each first air channel is formed between a portion of the outer circumferential surface of the light module and a portion of an inner surface of the housing of the disinfecting device. In these embodiments the first air channel is formed in a simple and practical way. The outer circumferential surface of the light module may, e.g., be configured to fit within the inner surface of the housing of the disinfecting device and may be provided with a recess at each location where a first air channel is to be provided. Alternatively, said inner surface of the housing of the disinfecting device may be provided with a recess at each location where a first air channel is to be provided.

In embodiments of the personal care system and the disinfecting device according to the invention, the housing of the disinfecting device comprises a central axis along which the receiving chamber, the light module and the air chamber are successively arranged, a first wall portion having a main direction of extension parallel to the central axis, and a second wall portion having a main direction of extension transverse to the central axis, wherein the air chamber is enclosed by the first wall portion, the second wall portion and the second main side of the light module, and wherein at least one of the second air channels is provided in the first wall portion. By providing the second air channel in said first wall portion, the natural-convection air flow in the air chamber may occur along a relatively large portion of the second main side of the light module, so that heating of the air flow at the second main side of the light module may be very effective. Preferably, the second air channel is provided in an area of the first wall portion where the first wall portion connects to the second wall portion.

In further embodiments of the personal care system and the disinfecting device according to the invention, the receiving chamber of the disinfecting device comprises a receiving opening via which said portion of the personal care treatment unit is receivable by the receiving chamber, the receiving opening is surrounded by an edge portion of the housing of the disinfecting device, and, in the disinfecting position of the personal care treatment unit, at least one of the third air channels is formed between a portion of the edge portion of the housing and a portion of an outer surface of the personal care treatment unit. In these embodiments the third air channel is formed in a simple and practical way. The outer surface of the personal care treatment unit may, e.g., be configured to fit within said edge portion of the housing of the disinfecting device and said edge portion may be provided with a recess at each location where a third air channel is to be provided. It is however noted that the invention also covers embodiments wherein the third air channel is formed in a different way, e.g., as a channel provided at a distance from the receiving opening in a wall portion of the housing of the disinfecting device that surrounds the receiving chamber.

In preferred embodiments of the personal care system and the disinfecting device according to the invention, the housing of the disinfecting device is configured as a protective covering member configured to be releasably placeable onto the personal care device to cover at least said portion of the personal care treatment unit. In these preferred embodiments the disinfecting device is configured as a protective cover for the personal care treatment unit, which is easily placeable by the user onto the personal care device. The disinfecting device may be provided with a timer to automatically deactivate the at least one light source of the light module after a predefined period of time so that, after the disinfecting process, the disinfecting device may stay in place on the personal care device to protect the personal care treatment unit. It is noted that the invention also covers embodiments wherein the disinfecting device is configured or integrated in a different way. The disinfecting device may, e.g., be integrated within or part of a charging device for the personal care device or be integrated within or part of a storage box or case configured to entirely contain and envelop the personal care device.

In embodiments of the personal care system and the disinfecting device according to the invention, the personal care device is an electric shaver and the personal care treatment unit is a shaving unit having at least one hair-cutting unit. Electric shavers are generally intended to shave beard hairs on the face of a human. Electric shavers may be of a rotary type, wherein each hair-cutting unit has an external cutting member with hair-entry openings and an internal cutting member with cutting elements covered by and rotatable within the external cutting member. Electric shavers may also be of a linearly reciprocating type, wherein each hair-cutting unit has an external cutting member with hair-entry openings and an internal cutting member with cutting elements covered by and linearly reciprocating within the external cutting member. The invention also covers embodiments wherein the personal care device is of a different type such as, e.g., a hair-trimming device intended to cut beard hairs on the face of a human or hairs on human body parts other than the face, intense pulsed light (IPL) devices intended to remove hairs on the legs or other body parts of a human by means of light pulses, facial-cleansing brushing devices intended to clean the face of a human by means of a rotating and/or vibrating brush, toothbrushes intended to clean the teeth of a human, and skin rejuvenation devices intended to rejuvenate the skin of body parts of a human by means of electromagnetic or radiofrequency energy.

The above-described and other aspects of the invention will be apparent from and elucidated with reference to the following detailed description of embodiments of a personal care system and a disinfecting device in accordance with the invention.

### BRIEF DESCRIPTION OF THE DRAWINGS

The invention will be explained in greater detail with reference to the figures, in which equal or similar features are indicated by the same reference signs, and in which:
Fig. 1 shows a personal care system according to the invention;
Fig. 2a shows a partial cross section of the personal care system along the line II-II in Fig. 1 in a first orientation of the personal care system;
Fig. 2b shows the cross section of Fig. 2a in a second orientation of the personal care system;
Fig. 3 shows a disinfecting device according to the invention which is part of the personal care system of Fig. 1;
Fig. 4 shows a cross section of the disinfecting device along the line IV-IV in Fig. 3;
Fig. 5 shows a cross section of the disinfecting device along the lines V-V in Fig. 1 and Fig. 3;
Fig. 6 shows a cross section of the personal care system along the line VI-VI in Fig. 1;
Fig. 7a shows a partial cross section of the personal care system along the line VII-VII in Fig. 2a in a first orientation of the personal care system; and
Fig. 7b shows the cross section of Fig. 7a in a second orientation of the personal care system.

### DETAILED DESCRIPTION OF THE EMBODIMENTS

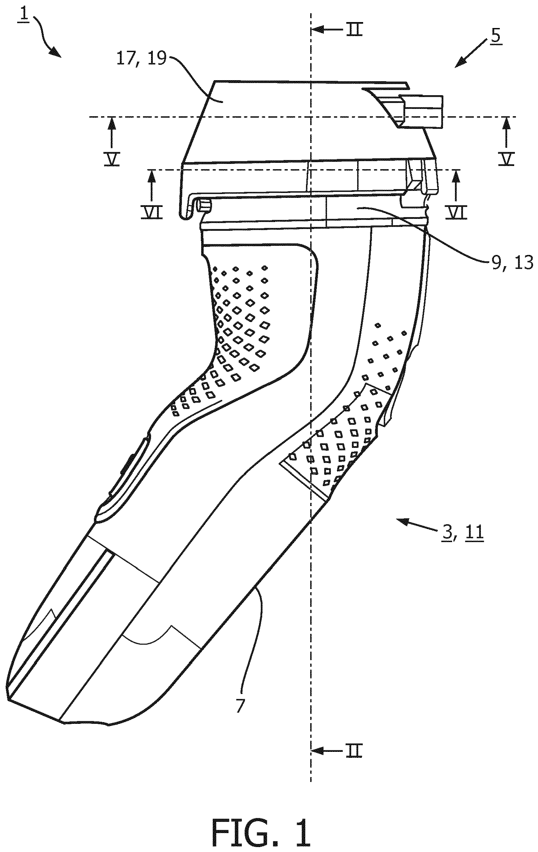

Fig. 1 shows a personal care system 1 according to the invention. The personal care system 1 comprises a personal care device 3 and a disinfecting device 5 according to the invention. The personal care device 3 comprises a main body 7 and a personal care treatment unit 9 arranged on the main body 7. The main body 7 is intended to be held by a hand of a user during use of the personal care device 3. The personal care treatment unit 9 is configured to provide a personal care treatment to a body part of the user. Fig. 1 shows the personal care system 1 in a condition wherein the disinfecting device 5 is placed on the personal care device 3 to disinfect the personal care treatment unit 9.

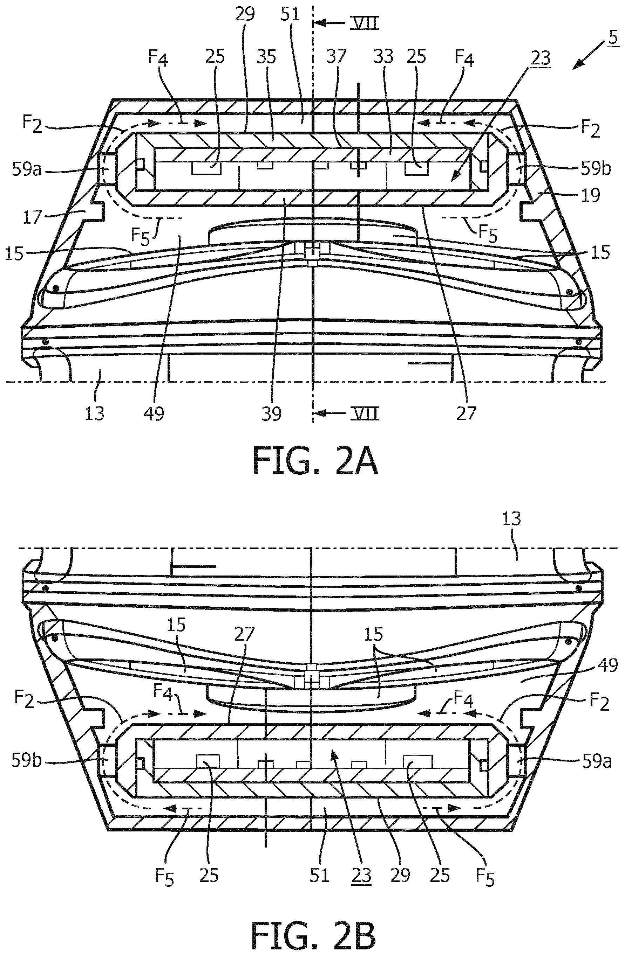

In the embodiment shown in Fig. 1, the personal care device 3 is an electric shaver 11 and the personal care treatment unit 9 is a shaving unit 13 having three hair-cutting units 15 of a rotary type. The hair-cutting units 15 are partially visible in Fig. 2a. Electric shavers with hair-cutting units of a rotary type are well known to the person skilled in the art. Therefore, the electric shaver 11, the shaving unit 13 and the hair-cutting units 15 will not be described in further detail. It is noted that the invention also covers embodiments wherein the personal care device is an electric shaver of a linearly reciprocating type. The invention also covers embodiments wherein the personal care device is of a type different from an electric shaver such as, e.g., a hair-trimming device, a non-electric hair-cutting device such as a blade razor, an intense pulsed light (IPL) device, a brushing device for facial cleansing, a toothbrush, or a light-based or radiofrequency energy-based skin rejuvenation device. All these types of personal care devices are well known to the person skilled in the art.

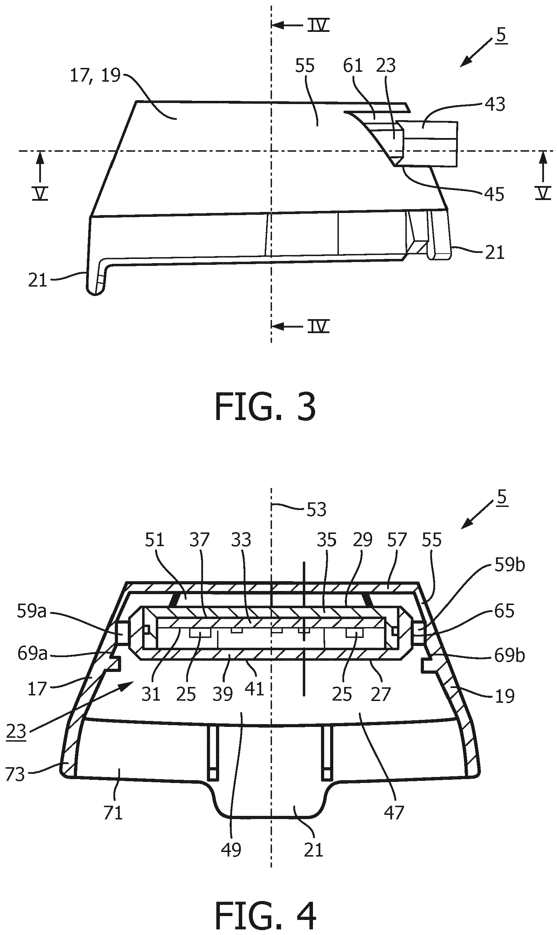

The disinfecting device 5 is configured to disinfect the shaving unit 13, in particular the three hair-cutting units 15 thereof, by means of disinfecting light. In embodiments of the invention, wherein the personal care device is of a type different from the electric shaver 11 as mentioned here before, the disinfecting device is configured to disinfect at least a portion of the personal care treatment unit of the personal care device. In the embodiment shown in Fig. 1, the disinfecting device 5 comprises a housing 17 which is configured as a protective covering member 19 configured to be releasably placeable on the electric shaver 11 to cover at least a portion of the shaving unit 13 including the three hair-cutting units 15. As shown in Fig. 3, which shows the disinfecting device 5 separated from the electric shaver 11, the covering member 19 comprises snap connection elements 21 by means of which the disinfecting device 5 is releasably connectable to the shaving unit 13. A user may thus easily place and connect the disinfecting device 5 onto the shaving unit 13 and use the disinfecting device 5 as a protective covering member 19 for the shaving unit 13 when the electric shaver 11 is not in use. To remove the disinfecting device 5, the user may easily pull the disinfecting device 5 off from the shaving unit 13, whereby the snap connection elements 21 are released.

As shown in Fig. 4, the disinfecting device 5 comprises a light module 23 which is arranged within the housing 17 of the disinfecting device 5. The light module 23 comprises a plurality of LEDs 25 configured to generate the disinfecting light. Instead of the LEDs 25, the light module 23 may have at least one light source of a different type suitable to generate the disinfecting light. The light module 23 has a first main side 27, via which during use the disinfecting light generated by the LEDs 25 is emitted, and a second main side 29 opposite to the first main side 27. In the embodiment shown in Fig. 4, the LEDs 25 are mounted on a first main surface 31 of a plate-shaped carrier 33, said first main surface 31 facing the first main side 27 of the light module 23. In the embodiment shown in Fig. 4, the plate-shaped carrier 33 is a printed circuit board, PCB. However, instead of the PCB, any suitable carrier for the LEDs 25 may be used. The light module 23 further comprises a metal heat sink 35 connected to a second main surface 37 of the carrier 33 opposite to the first main surface 31 of the carrier 33.

As shown in Fig. 4, the light module 23 further comprises a plate-shaped light-diffusing member 39 which is arranged at the first main side 27 of the light module 23. The plate-shaped light-diffusing member 39 is made from an optically transparent diffusive material and extends parallel to the first main side 27 of the light module 23. In the embodiment shown in Fig. 4, the first main side 27 of the light module 23 is formed by a main side 41 of the plate-shaped light-diffusing member 39 facing away from the carrier 33. Said main side 41 of the plate-shaped light-diffusing member 39 may be provided with an optical structure, not shown in the figures, comprising a plurality of facets. Alternatively and even preferably, such optical structure may be provided on a main side of the plate-shaped light-diffusing member 39 opposite to the main side 41 and facing the LEDs 25.

As shown in Fig. 3, the light module 23 further comprises an electric connector 43 by means of which the light module 23 may be connected to an external power source not shown in the figures. The electric connector 43 may be of any suitable type, such as a so-called pig-nose connector or a USB connector. As shown in Fig. 3, the light module 23 is mounted to the housing 17 of the disinfecting device 5 mainly by means of a supporting member 45 of the housing 17.

In the embodiments of the personal care system 1 and the disinfecting device 5 as shown in the figures, the disinfecting light generated by the LEDs 25 has a peak wavelength in a range from 400 to 425 nm, more preferably in a range from 405 to 410 nm. I.e., the peak wavelength of the disinfecting light generated by the LEDs 25 is in the violet range of visible light. Although the effectiveness of violet light for reducing bacteria is less than the effectiveness of UV light for reducing bacteria, the effectiveness of violet light appeared to be sufficiently high for the disinfecting purposes of the disinfecting device 5. In particular, with violet light having a peak wavelength within the range from 405 to 410 nm as used in the present embodiments, a 99.9% reduction of Escherichia coli, E. coli, and Staphylococcus aureus, S. aureus, bacteria could be achieved within 2 hours of exposure of the shaving unit 13 to the disinfecting light.

An advantage of using violet light is that violet light is significantly less harmful for the user than UV light. In particular in combination with the light-diffusing member 39, the peak irradiances and peak radiances of the violet disinfecting light emitted at the first main side 27 of the light module 23 are sufficiently reduced to prevent damage to the user's eyes and skin in case of an unintentional exposure of the user to the violet disinfecting light emitted at the first main side 27 of the light module 23. In this respect, the light-diffusing member 39 increases the angular distribution of the disinfecting light generated by the LEDs 25 as the disinfecting light passes the light-diffusing member 39. Thereby, the light-distributing member 39 distributes the disinfecting light to a higher degree over the shaving unit 13 and the hair-cutting units 15 thereof, thus improving uniformity of irradiance. In addition, the increased angular distribution of the disinfecting light implies a lower peak radiance and, therefore, helps to prevent eye damage. As a result, any safety measures to be provided in the disinfecting device 5 to prevent unintentional exposure of the user to the disinfecting light are less stringent as compared with disinfecting devices that use UV light. In particular, the disinfecting device 5 may just have a switch, not shown in the figures and operable by the user to activate the LEDs 25, and a timer configured to automatically deactivate the LEDs 25 after a predetermined time period, e.g., after two hours, when the disinfecting process is considered to be completed. An additional safety system, to automatically deactivate the LEDs 25 in case the disinfecting device 5 is removed from the shaving unit 13 and the LEDs 25 are still active, may not be required.

As shown in Fig. 4, the disinfecting device 5 further comprises a receiving space 47 arranged at the first main side 27 of the light module 23 and configured to receive at least a portion of the shaving unit 13 including the three hair-cutting units 15. The receiving space 47 is provided in a receiving chamber 49 which is arranged in the housing 17 of the disinfecting device 5. The receiving chamber 49 is configured to hold the shaving unit 13 in a disinfecting position within the receiving chamber 49, as shown in Fig. 2a.

As shown in Fig. 4, the disinfecting device 5 further comprises an air chamber 51 arranged in the housing 17 of the disinfecting device 5 at the second main side 29 of the light module 23. The receiving chamber 49, the light module 23 and the air chamber 51 are successively arranged along a central axis 53 of the housing 17 of the disinfecting device 5. The air chamber 51 is enclosed by the second main side 29 of the light module 23, a first wall portion 55 of the housing 17 having a main direction of extension parallel to the central axis 53, and a second wall portion 57 of the housing 17 having a main direction of extension transverse to the central axis 53.

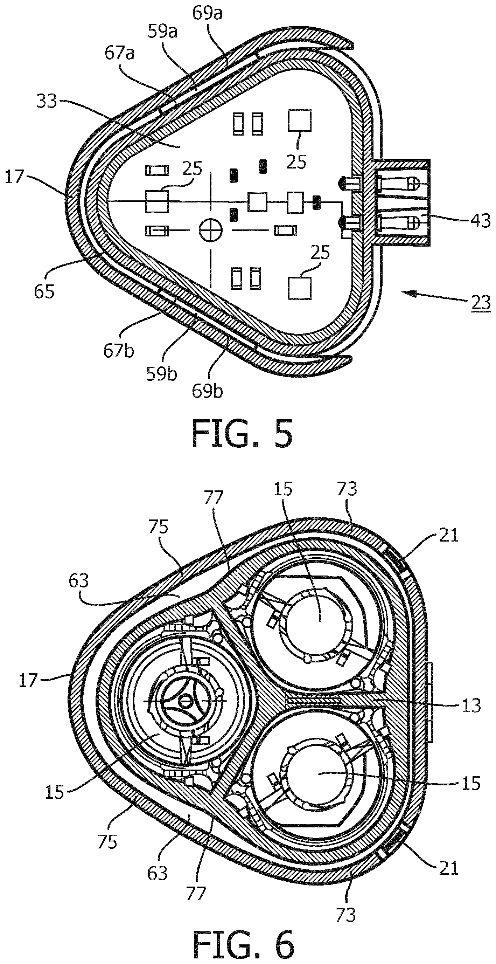

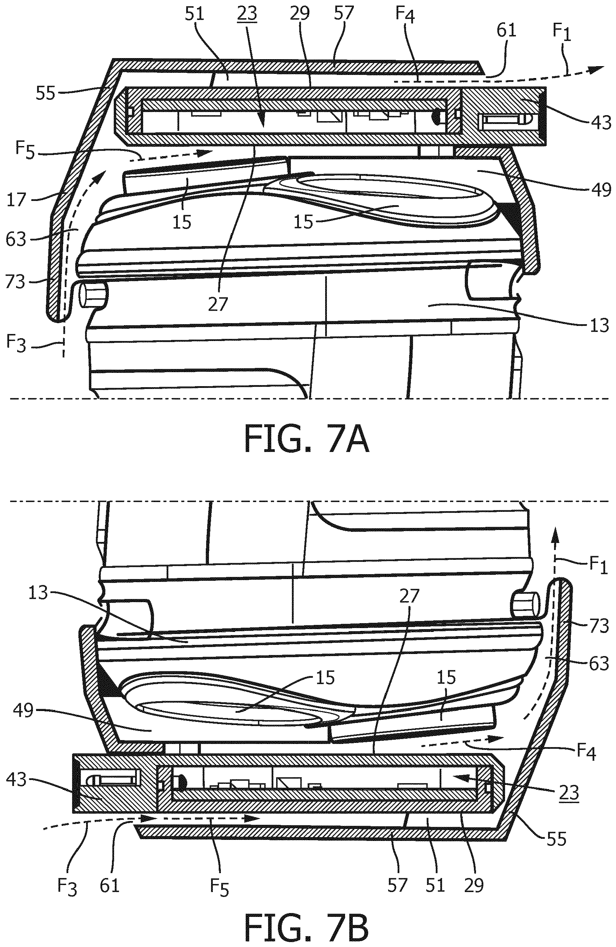

According to the invention, the disinfecting device 5 comprises at least one first air channel 59a, 59b which connects the receiving space 47 of the receiving chamber 49 with the air chamber 51, and at least one second air channel 61 which connects the air chamber 51 with the ambient space surrounding the housing 17. The disinfecting device 5 is further configured to provide, when the shaving unit 13 is in the disinfecting position within the receiving chamber 49 as shown in Fig. 2a, at least one third air channel 63 (see Figs. 6 and 7a) which connects the receiving chamber 49 with the ambient space surrounding the housing 17.

As shown in Fig. 4 and Fig. 5, the light module 23 comprises an outer circumferential surface 65 which extends between the first main side 27 and the second main side 29 of the light module 23. In the embodiment shown in the figures, two first air channels 59a, 59b are provided in mutually opposite positions relative to the light module 23. As shown in Fig. 5, the first air channels 59a, 59b are each formed between a respective one of two portions 67a, 67b of the outer circumferential surface 65 of the light module 23 and a respective one of two recessed portions 69a, 69b of an inner surface of the housing 17 of the disinfecting device 5. Thus, in accordance with the invention, the first air channels 59a, 59b are each arranged exclusively outside of the light module 23.

In the embodiment shown in the figures and as particularly shown in Fig. 3 and Fig. 7a, the second air channel 61 is provided as an opening in the first wall portion 55 of the housing 17. Said opening also serves to make the electric connector 43 accessible for the user.

As shown in Fig. 4, the receiving chamber 49 of the disinfecting device 5 comprises a receiving opening 71 via which the shaving unit 13 is receivable by the receiving chamber 49. The receiving opening 71 is situated opposite to the first main side 27 of the light module 23 and is surrounded by an edge portion 73 of the housing 17 of the disinfecting device 5. As shown in Fig. 6, when the shaving unit 13 is in the disinfecting position within the receiving chamber 49 (as shown in Fig. 2a), the third air channel 63 is formed as a gap between a portion 75 of the edge portion 73 of the housing 17 and a portion 77 of an outer surface of the shaving unit 13.

During use of the disinfection device 5, i.e., with the shaving unit 13 being arranged in the disinfecting position within the receiving chamber 49 as shown in Fig. 2a and with the LEDs 25 of the light module 23 being activated, the violet disinfecting light generated by the LEDs 25 will be emitted via the first main side 27 of the light module 23 towards the shaving unit 13 to disinfect the shaving unit 13 and in particular the hair-cutting units 15 thereof. At the same time, the LEDs 25 will generate heat as a result of the limited electrical-to-optical energy conversion efficiency of the LEDs 25. A largest portion of the heat generated by the LEDs 25 will be absorbed by the heat sink 35, which is arranged at the second main side 29 of the light module 23 and connected to the second main surface 37 of the carrier 33. A smaller portion of the heat generated by the LEDs 25 will be absorbed by the light-diffusing member 39 arranged at the first main side 27 of the light module 23. As a result, both the air which is present in the air chamber 51 and the air which is present in the receiving chamber 49 will be heated by the heat generated by the LEDs 25, i.e., via respectively the heat sink 35 and the light-diffusing member 39. The air in the air chamber 51 will however be heated more than the air in the receiving chamber 49. In the following, the result of the heating of the air in the air chamber 51 and the receiving chamber 49 will be described for two different orientations of the personal care system 1, i.e., a first orientation as shown in Fig. 7a wherein the electric shaver 11 is in a upright vertical orientation (as shown in Fig. 1) and wherein, accordingly, the air chamber 51 is positioned above the receiving chamber 49, and a second orientation as shown in Fig. 7b wherein the electric shaver 11 is in an upside-down vertical orientation and wherein, accordingly, the air chamber 51 is positioned below the receiving chamber 49.

In said first orientation as shown in Fig. 7a, the heated air in both the air chamber 51 and the receiving chamber 49 will expand. Said expansion of the air in the air chamber 51 will result in an air flow F₁ from the air chamber 51 to the ambient space via the second air channel 61, as shown in Fig. 7a. The resulting decreasing pressure in the air chamber 51 will result in an air flow F₂ from the receiving chamber 49 to the air chamber 51 via the first air channels 59a, 59b, as shown in Fig. 2a. As a result, a flow F₃ of fresh ambient air will be generated from the ambient space into the receiving chamber 49 via the third air channel 63, as shown in Fig. 7a. Thus, the heating of the air in the air chamber 51 results in an overall air flow through the disinfecting device 5 from the ambient space via, respectively, the third air channel 63, the receiving chamber 49, the first air channels 59a, 59b, the air chamber 51 and the second air channel 61 back into the ambient space. The parts of said overall air flow within the air chamber 51 and within the receiving chamber 49 are indicated by, respectively, F₄ and F₅ in Fig. 2a and Fig. 7a.

The heated air in both the air chamber 51 and the receiving chamber 49 will expand also in said second orientation as shown in Fig. 7b. Said expansion of the air in the receiving chamber 49 will result in an air flow F₁ from the receiving chamber 49 to the ambient space via the third air channel 63, as shown in Fig. 7b. The resulting decreasing pressure in the receiving chamber 49 will result in an air flow F₂ from the air chamber 51 to the receiving chamber 49 via the first air channels 59a, 59b, as shown in Fig. 2b. Said air flow F₂ from the air chamber 51 to the receiving chamber 49 is further increased by said expansion of the air in the air chamber 51. As a result, a flow F₃ of fresh ambient air will be generated from the ambient space into the air chamber 51 via the second air channel 61, as shown in Fig. 7b. Thus, the heating of the air in the air chamber 51 and the receiving chamber 49 results in an overall air flow through the disinfecting device 5 from the ambient space via, respectively, the second air channel 61, the air chamber 51, the first air channels 59a, 59b, the receiving chamber 49 and the third air channel 63 back into the ambient space. The parts of said overall air flow within the receiving chamber 49 and within the air chamber 51 are indicated by, respectively, F₄ and F₅ in Fig. 2b and Fig. 7b.

Thus, both in the first orientation of the personal care system 1 shown in Figs. 1, 2a and 7a and in the second orientation of the personal care system 1 shown in Figs. 2b and 7b an air flow, with air flow components F₁, F₂, F₃, F₄, F₅, is generated in the disinfecting device 5 which is only based on natural convection, i.e., without the use of forced convection by a fan, pump, suction system or similar means. Because, according to the invention, the first air channels 59a, 59b, that connect the air chamber 51 with the receiving chamber 49, are arranged outside of the light module 23, a relatively long flow path is created for said natural-convection air flow along the first and second main sides 27, 29 of the light module 23 in both said first and said second orientation of personal care system 1, which results in an effective heating of the air flows F₄, F₅ in the air chamber 51 and the receiving chamber 49 and, consequently, a relatively strong overall natural-convection air flow through the disinfecting device 5 and, in particular, along the shaving unit 13. In cases wherein the shaving unit 13 is placed into the receiving chamber 49 in a wet condition, e.g., after cleaning of the shaving unit 13 under a water tap, said natural-convection air flow along the shaving unit 13 appeared to be sufficiently strong to dry the shaving unit 13 within a reasonable time, e.g., within 1 hour. In said first orientation of the personal care system 1, drying of the shaving unit 13 will be caused by relatively cold ambient air flowing into the receiving chamber 49 by the air flow F₃ via the third air channel 63, as shown in Fig. 7a. In said second orientation of the personal care system 1, drying of the shaving unit 13 will be caused even more efficiently by relatively warm air flowing into the receiving chamber 49 by the air flow F₂ via the first air channels 59a, 59b, as shown in Fig. 2b.

The natural-convection air flow, with the air flow components F₁, F₂, F₃, F₄, F₅, through the disinfecting device 5 will also efficiently cool the light module 23. Furthermore, the air chamber 51 thermally isolates the first wall portion 55 and the second wall portion 57 of the housing 17 relative to the light module 23, so that the risk that a user will be exposed to high temperatures when touching the housing 17 of the disinfecting device 5 is significantly reduced.

The flow path of the air flow in the air chamber 51 (F₄ in the first orientation and F₅ in the second orientation of the personal care system 1) will depend on the number and positions of the first air channels 59a, 59b and the second air channels 61 relative to the air chamber 51. The flow path of the air flow in the receiving chamber 49 (F₅ in the first orientation and F₄ in the second orientation of the personal care system 1) will depend on the number and positions of the first air channels 59a, 59b and the third air channels 63 relative to the receiving chamber 49. It will be clear for the skilled person that the number and positions of the first, second and third air channels can be selected in a straight-forward manner to create a desired flow-path length in the air chamber 51 and in the receiving chamber 49. For example, in the embodiments of the disinfecting device 5 shown in the figures, the second air channel 61 is provided in the first wall portion 55 of the housing 17, so that the natural-convection air flow F₄, F₅ in the air chamber 51 may occur along a relatively large portion of the second main side 29 of the light module 23 and, consequently, heating of the air flow F₄, F₅ at the second main side 29 of the light module 23 may be very effective. However, in alternative embodiments one or more second air channels may be provided in the second wall portion 57 of the housing 17, but with this alternative position of the second air channels the heating of the air in the air chamber 51 may be less efficient. Likewise, in alternative embodiments the number and positions of the first and third air channels may be different from the number and positions of the first air channels 59a, 59b and the third air channel 63 shown in the figures. One or more third air channels may, e.g., be provided at a distance from the receiving opening 71 in a wall portion of the housing 17 of the disinfecting device 5 that surrounds the receiving chamber 49.

In a personal care system and a disinfecting device in accordance with the invention the first, second and third air channels may also be used as flushing channels for a flushing liquid to clean the personal care treatment unit of the personal care device. For example, in the embodiments shown in figures, when the disinfecting device 5 is placed on the shaving unit 13, a user may hold the electric shaver 11 such that the disinfecting device 5 is flooded by a flow of tap water. In this way, a flow of tap water may be created through the receiving chamber 49 and the air chamber 51 via the first, second and third air channels to clean the shaving unit 13 by means of the tap water.

Variations to the disclosed embodiments can be understood and effected by those skilled in the art in practicing the principles and techniques described herein, from a study of the drawings, the disclosure and the appended claims. In the claims, the word "comprising" does not exclude other elements or steps, and the indefinite article "a" or "an" does not exclude a plurality. The mere fact that certain measures are recited in mutually different dependent claims does not indicate that a combination of these measures cannot be used to advantage. Any reference signs in the claims should not be construed as limiting the scope of the claims.

## Claims

1. A personal care system (1) comprising a personal care device (3) and a disinfecting device (5), wherein:
- the personal care device comprises a personal care treatment unit (9) configured to provide a personal care treatment to a body part of a subject; and
- the disinfecting device is configured to disinfect the personal care treatment unit by means of disinfecting light;
and wherein the disinfecting device comprises:
- a housing (17);
- a light module (23) arranged in the housing and comprising at least one light source (25) configured to generate the disinfecting light, a first main side (27) via which during use the disinfecting light generated by the at least one light source is emitted, and a second main side (29) opposite to the first main side;
- a receiving space (47) arranged at the first main side of the light module and configured to receive at least a portion of the personal care treatment unit;
- an air chamber (51) arranged in the housing at the second main side of the light module;
- at least one first air channel (59a, 59b) connecting the receiving space with the air chamber; and
- at least one second air channel (61) connecting the air chamber with ambient space surrounding the housing;
**characterized in that:**
- the receiving space (47) is provided in a receiving chamber (49) which is arranged in the housing (17) of the disinfecting device (5) and configured to hold said portion of the personal care treatment unit (9) in a disinfecting position within the receiving chamber;
- each first air channel (59a, 59b) is arranged exclusively outside of the light module (23); and
- in the disinfecting position of the personal care treatment unit, at least one third air channel (63) is provided connecting the receiving chamber with the ambient space surrounding the housing.

2. A disinfecting device (5) configured to disinfect a personal care treatment unit (9) of a personal care device (3) by means of disinfecting light, wherein the personal care treatment unit is configured to provide a personal care treatment to a body part of a subject; wherein the disinfecting device comprises:
- a housing (17);
- a light module (23) arranged in the housing and comprising at least one light source (25) configured to generate the disinfecting light, a first main side (27) via which during use the disinfecting light generated by the at least one light source is emitted, and a second main side (29) opposite to the first main side;
- a receiving space (47) arranged at the first main side of the light module and configured to receive at least a portion of the personal care treatment unit;
- an air chamber (51) arranged in the housing at the second main side of the light module;
- at least one first air channel (59a, 59b) connecting the receiving space with the air chamber; and
- at least one second air channel (61) connecting the air chamber with ambient space surrounding the housing;
**characterized in that:**
- the receiving space (47) is provided in a receiving chamber (49) which is arranged in the housing (17) of the disinfecting device (5) and configured to hold said portion of the personal care treatment unit (9) in a disinfecting position within the receiving chamber;
- each first air channel (59a, 59b) is arranged exclusively outside of the light module (23); and
- the disinfecting device is configured to provide, in the disinfecting position of the personal care treatment unit, at least one third air channel (63) connecting the receiving chamber with the ambient space surrounding the housing.

3. The personal care system (1) as claimed in claim 1, or the disinfecting device (5) as claimed in claim 2, wherein:
- the light module (23) comprises a carrier (33);
- the at least one light source (25) is mounted on a first main surface (31) of the carrier which faces the first main side (27) of the light module; and
- the light module comprises a heat sink (35) connected to a second main surface (37) of the carrier opposite to the first main surface of the carrier.

4. The personal care system (1) as claimed in claim 1 or 3, or the disinfecting device (5) as claimed in claim 2 or 3, wherein the light module (23) comprises a light-diffusing member (39) arranged at the first main side (27) of the light module (23).

5. The personal care system (1) as claimed in any of claims 1, 3 or 4, or the disinfecting device (5) as claimed in any of claims 2-4, wherein the disinfecting light generated by the at least one light source (25) has a peak wavelength in a range from 400 to 425 nm, preferably in a range from 405 to 410 nm.

6. The personal care system (1) as claimed in any of claims 1 or 3-5, or the disinfecting device (5) as claimed in any of claims 2-5, wherein:
- the light module (23) comprises an outer circumferential surface (65) extending between the first and second main sides (27, 29) of the light module; and
- each first air channel (59a, 59b) is formed between a portion (67a, 67b) of the outer circumferential surface of the light module and a portion (69a, 69b) of an inner surface of the housing (17) of the disinfecting device (5).

7. The personal care system (1) as claimed in any of claims 1 or 3-6, or the disinfecting device (5) as claimed in any of claims 2-6, wherein the housing (17) of the disinfecting device (5) comprises:
- a central axis (53) along which the receiving chamber (49), the light module (23) and the air chamber (51) are successively arranged;
- a first wall portion (55) having a main direction of extension parallel to the central axis; and
- a second wall portion (57) having a main direction of extension transverse to the central axis;
and wherein:
- the air chamber is enclosed by the first wall portion, the second wall portion and the second main side (29) of the light module; and
- at least one of the second air channels (61) is provided in the first wall portion.

8. The personal care system (1) as claimed in any of claims 1 or 3-7, or the disinfecting device (5) as claimed in any of claims 2-7, wherein:
- the receiving chamber (49) of the disinfecting device (5) comprises a receiving opening (71) via which said portion of the personal care treatment unit (9) is receivable by the receiving chamber;
- the receiving opening is surrounded by an edge portion (73) of the housing (17) of the disinfecting device; and
- in the disinfecting position of the personal care treatment unit, at least one of the third air channels (63) is formed between a portion (75) of the edge portion of the housing and a portion (77) of an outer surface of the personal care treatment unit.

9. The personal care system (1) as claimed in any of claims 1 or 3-8, or the disinfecting device (5) as claimed in any of claims 2-8, wherein the housing (17) of the disinfecting device (5) is configured as a protective covering member (19) configured to be releasably placeable onto the personal care device (3) to cover at least said portion of the personal care treatment unit (9).

10. The personal care system (1) as claimed in any of claims 1 or 3-9, or the disinfecting device (5) as claimed in any of claims 2-9, wherein the personal care device (3) is an electric shaver (11), and wherein the personal care treatment unit (9) is a shaving unit (13) having at least one hair-cutting unit (15).
